# EUROPEAN PATENT APPLICATION

(11) **EP 3 153 204 A1**
(43) Date of publication of application: **12.04.2017**
(21) Application number: 15803702.8
(22) Date of filing: 02.06.2015
(51) Int. Cl.: A61M 25/00

(54) **MEDICAL DEVICE**

(30) Priority: 03.06.2014 JP 2014114889; 03.06.2014 JP 2014114891
(71) Applicant: Sumitomo Bakelite Co., Ltd., Shinagawa-ku Tokyo 140-0002 (JP)
(72) Inventor: TOYOTA, Yoshiki, Akita-shi, Akita 011-8510 (JP); FUJITA, Yasuhiro, Akita-shi, Akita 011-8510 (JP)
(74) Representative: Müller-Boré & Partner Patentanwälte PartG mbB
(86) International application number: PCT/JP2015/065869
(87) International publication number: WO 2015/186692

(57) **Abstract**

The invention provides a medical device including a resin tube having excellent branch selectivity while excellent pushability in a curved body cavity or an extremely narrow body cavity such as a blood vessel terminal region is maintained. Provided is a medical device having a medical device body to be inserted into a body cavity. The medical device body has an elongated resin tube, the resin tube includes a first region including a distal end part and a second region including a proximal end part, and the first region is more flexible than the second region.

## Description

### Technical Field

The present invention relates to a medical device.

Priority is claimed on Japanese Patent Application Nos. 2014-114889 and 2014-114891, filed June 3, 2014, the content of which is incorporated herein by reference.

### Background Art

In recent years, development of catheters that can be inserted into body cavities, such as a blood vessel, has been performed. Generally, catheters are inserted into body cavities by an over-the-wire technique using a guide wire. The guide wire is retained in a bending state ahead of a desired branch passage, and a catheter covers the guide wire from a base end side of the guide wire and is pushed in along the guide wire. Accordingly, the branch passage can be selected. Since the desired branch passage is selected in a branch of the body cavity and the catheter proceeds further in an insertion direction, it is usual that a catheter is moderately flexibly formed. Therefore, it is usual that catheters have a resin tube that is elongated and has flexibility. Particularly, catheters including a resin tube that can also be applied to narrow body cavities in which branches are intricately curved as in blood vessel terminal regions and enable a branch to be excellently selected have been suggested.

For example, a catheter (hereinafter referred to as Related Art 1) described in PTL 1 includes a resin outer layer in which a base end part and a tip part have different hardnesses, that is, flexibilities. Specifically, a resin tube that is the resin outer layer is formed using four kinds of polyether ester amide elastomer having different Shore D hardnesses. It is described in PTL 1 that the hardnesses of the above four kinds of polyether ester amide elastomer are proportional to weight ratio of hard segments of the polyether ester amide elastomers.

Additionally, elongated medical devices, such as the above catheters and puncturing needles for an endoscope, have common properties that these devices have flexibility and include an elongated sheath and a hub on a hand side. The sheath includes one or a plurality of inner holes (lumens) that extend from a tip of the sheath to a base end thereof, and items of various kinds, such as a medicinal solution, a contrast medium, a guide wire, a puncturing needle, and other medical devices, are supplied to the insides of the inner holes. As the sheath, a one-fold tube or a double-fold tube is used depending on the type of medical device.

It is usual that the hub is made of hard materials and has a greater diameter than the sheath so that so that handleability for the user becomes excellent. It is usual that the hub is hollow and communicates with the inner holes of the sheath, and, a base end of the hub is provided with a connector for allowing a syringe to be attached thereto.

With respect to this type of medical device, a medical device (catheter) including an elongated sheath (catheter body) made of a double-fold tube and a hub provided at a base end part of the sheath is described in PTL 2. The base end part of the sheath is inserted into and fixed to the hub, and holds the vicinity of this fixed part with a kink-resistant protector (protector). The protector is a tubular protecting member that prevents a situation in which the base end part of the sheath is bent and the inner holes become blocked (kinked), and is provided at a tip part of the hub. The protector is also referred to as a connector cover, a strain reliever, or the like, and has sufficient bending rigidity and hardness.

The sheath is made using a resin material, such as polyurethane-based resin or polyamide-based resin, as a main component. In order to improve visibility in a state where the sheath is placed on an instrument platform or a surgical bed, it is usual that the sheath is opaque and is colored in various colors. Meanwhile, medical devices adapted such that a sheath is made transparent and a medicinal solution passing through the sheath from the outside thereof can be visually checked from outside thereof are described in PTL 3 and PTL 4.

### Citation List

### Patent Literature

[PTL 1] Japanese Unexamined Patent Application, First Publication No. 2007-082802
[PTL 2] Japanese Unexamined Patent Application, First Publication No. 2012-223346
[PTL 3] Japanese Unexamined Patent Application, First Publication No. 2003-190275
[PTL 4] Japanese Unexamined Patent Application, First Publication No. 2006-110224

### Summary of Invention

### Technical Problem

Meanwhile, a catheter moves forward in a body cavity so as to be pushed in a forward movement direction along the guide wire, in a non-branch region, or in a branch passage of which the internal diameter is relatively large and the curving angle is gentle. Therefore, it is preferable that the catheter, also including a tip part thereof, has moderate rigidity to such a degree that excellent pushability is exhibited, in addition to slidability with respect to the guide wire. That is, the resin tube of the catheter requires moderate rigidity in order to have excellent pushability.

Meanwhile, in a case where the catheter has approached a region where branch selection is not easy, such as a branch passage of which the internal diameter is small and the curving angle is sharp, it is preferable that a tip part of the above resin tube is flexible in order to obtain excellent branch selectivity. Accordingly, the catheter can be inserted into a desired branch passage, with the bending state of the guide wire retained in the branch passage being maintained in the body cavity. However, if the tip is hard, when the catheter is pushed in along the guide wire, the tip part of the guide wire cannot be retained in the bending state in the desired branch passage, and branch selectivity may become poor.

In this way, catheters capable of responding to complex requirements also including the tip part, exhibiting excellent pushability and exhibiting flexibility to such a degree that the tip part of the catheter shows excellent branch selectivity in a branch passage have not yet suggested.

Additionally, in a case where this type of medical device is inserted into a body cavity, it is usual to retain a guiding catheter (master catheter) or an endoscope in a subject and insert the medical device from a connector or a proximal operating part at a proximal end part of the guiding catheter or the endoscope. In a case where the medical device is deeply inserted into a body cavity, the hub of the medical device may reach the vicinity of the connector of the guiding catheter or the proximal operating part (hereinafter may be referred to as a body cavity inlet) of the endoscope.

Here, as previously described, there may be a medical device in which the hub is made of a transparent resin material and a tip of the syringe filled with a medicinal solution being normally mounted can be visually checked. In contrast, the protector is formed of an opaque resin material, and thereby the visibility of the medical device in a state where the medical device is placed on an instrument platform or a surgical bed is secured.

Such a medical device requires that it is visually checked that a medicinal solution is normally supplied from the syringe to the sheath and flows toward the tip side and it can be rapidly seen that a subject's blood flows in from a tip opening of the sheath and flows toward the base end side of the sheath (reverse flow blood). Even if the sheath is made transparent as in PTL 2 or PTL 3, if the sheath is deeply inserted into a body cavity until the hub and the protector approach a body cavity inlet, it is impossible to visually recognize the insides of the inner holes through a wall surface of the sheath. For this reason, when the sheath is deeply inserted into a body cavity, a tip of the sheath is made to reach the vicinity of a target living body tissue, and subsequently, a medicinal solution is discharged or various kinds of procedure operations are performed, there is a problem that the flow of the medicinal solution or generation of reverse flow blood cannot be visually checked.

The invention has been made in view of the above problems. That is, an object of the invention is to provide a medical device including a resin tube allowing excellent branch selectivity while excellent pushability in a curved body cavity or an extremely narrow body cavity such as a blood vessel terminal region is maintained.

Moreover, an object of the invention is to provide a medical device that has the above excellent pushability and branch selectivity and allows an internal state of an inner hole of a sheath to be seen and visually checked even in a state where the sheath is sufficiently deeply inserted into a body cavity.

### Solution to Problem

In order to solve the above problems, the invention provides the following medical devices (1) to (19).
(1) A medical device including a medical device body to be inserted into a body cavity, the medical device body includes an elongated resin tube, the resin tube including a first region including an insertion-tip-side end part and a second region including a proximal end part, and the first region being more flexible than the second region.
(2) The medical device described in (1) in which the first region is formed of a first resin member, the second region is formed of a resin member different from the first resin member, and a difference X between a resin hardness of the first resin member measured at normal temperature and a resin hardness of the first resin member measured after being left for a predetermined time in a temperature environment of 38°C is greater than a difference Y between a resin hardness of the second resin member measured at the normal temperature, and the resin hardness of the second resin member measured after being left for the predetermined time in the temperature environment of 38°C.
(3) The medical device described in (2) in which the first resin member contains 50% or more of a first resin, and the second resin member contains 50% or more of a second resin that is resin different from the first resin.
(4) The medical device described in (2) or (3) in which the first resin is thermoplastic polyurethane-based resin, and the second resin is polyamide-based resin.
(5) The medical device described in any of (2) to (4) in which, in the first resin member and the second resin member, (I) the resin hardness of the first resin member measured at the normal temperature is greater than the resin hardness of the second resin member, and (II) the resin hardness of the first resin member measured after being left for a predetermined time in the temperature environment of 38°C is smaller than the resin hardness of the second resin member.
(6) The medical device described in any of (2) to (5) in which the medical device further includes an inner tube stacked on an inner periphery of the resin tube, a tip of the inner tube being located at the same position as a tip of the first region in a direction of a central axis.
(7) The medical device described in any of (2) to (6) in which a tip of the first region constitutes a tip of the resin tube.
(8) The medical device described in any one of (2) to (7) in which a marker that is provided at a distal end part and includes an X-ray impermeable metallic material is covered with the first resin member.
(9) The medical device described in any of (2) to (8), in which the medical device further includes a metal protective layer provided on an inner peripheral side of the resin tube or inside the resin tube, a tip of the metal protective layer being closer to a base-end-side end part than to the tip of the first region is in a direction of a central axis.
(10) The medical device described in any of (2) to (9) in which the dimension of the first region in the direction of the central axis is 5 mm or greater to 15 mm or smaller.
(11) The medical device described in (1) in which a metal protective layer formed by braiding wires is provided inside at least the second region of the resin tube, and an opening area ratio of the metal protective layer in a base-end-side end part of the resin tube exceeds 50%.
(12) The device described in (11) in which the metal protective layer is provided in the first region and the second region of the resin tube, and the opening area ratio of the metal protective layer in the base-end-side end part of the second region is greater than the opening area ratio in an insertion-tip-side end part of the first region.
(13) The medical device described in (11) or (12) in which the pitch of the wires becomes gradually narrower over the second region to the first region.
(14) The medical device described in (1), in which the medical device further includes a hollow hub provided at a base-end-side end part of the resin tube so as to communicate with an inner hole of the resin tube and a protector provided at a tip part of the hub to hold an outer periphery of the base-end-side end part of the resin tube, both the protector and a holding region held by the protector in the base-end-side end part of the resin tube having visible light permeability, and an internal state of the inner hole being visually recognizable.
(15) The medical device described in (14) in which an extending region that extends closer to a tip side than to the protector in the base-end-side end part of the resin tube has visible light permeability, and a tip part closer to the tip side than to the extending region, in the resin tube, has visible light impermeability.
(16) The medical device described in (15) in which the holding region and the extending region of the resin tube are translucent.
(17) The medical device described in (15) or (16) in which the outer diameter of the resin tube is the same over the extending region and the tip part of the resin tube, and the tip part of the resin tube is adapted such that the outer diameter of the resin tube decreases toward a distal end of the resin tube.
(18) The medical device described in any of (15) to (17) in which a hydrophilic coat layer is provided on the outer surface of the resin tube over the insertion-tip-side end part to the extending region, and the hydrophilic coat layer terminates in the middle of the extending region.
(19) The medical device described in any of (1) to (18) in which the medical device is a catheter.

### Advantageous Effects of Invention

The invention includes the resin tube in which the tip part is more flexible than the base end part under predetermined conditions. Accordingly, in the medical device of the invention, branch selectivity is excellent while excellent pushability in extremely narrow body cavities is maintained, such as an intricately curved body cavity or a blood vessel terminal region.

According to the medical device of the invention, it is possible to visually check the internal state of the inner hole of the catheter body through the protector even in a state where a sheath (catheter body) is sufficiently deeply inserted into a body cavity, in addition to providing the above excellent effects. Accordingly, poor injection of a medicinal solution or generation of reverse flow blood can be rapidly ascertained even in a state where the tip of the catheter body has reached the vicinity a target living body tissue.

### Brief Description of Drawings

FIG. 1 is an overall view of a catheter in a first embodiment of the invention.
FIG. 2 is a longitudinal sectional view of a tip part of a catheter body that is a first embodiment.
FIG. 3 is an explanatory view of the tip part of the catheter body that is the first embodiment.
FIG. 4 is a side view illustrating a metal protective layer in a state where an outer layer is removed from the catheter body that is the first embodiment.
FIG. 5 is a side view illustrating a catheter related to a second embodiment of the invention.
FIG. 6 is a longitudinal sectional view illustrating the internal structure of a first region of a catheter body in the second embodiment.
FIG. 7 is an enlarged view schematically illustrating a second region (extending region) of the catheter body in the second embodiment.
FIG. 8 is an enlarged view schematically illustrating the first region of the catheter body in the second embodiment.

### Description of Embodiments

Hereinafter, a first embodiment of the invention will be described with reference to the drawings. In addition, in all the drawings, the same constituent elements will be designated by the same reference numerals, and duplicate description will be appropriately omitted.

Various constituent elements of a medical device of the invention do not need to be individually and independently present, and the invention allows that a plurality of constituent elements are formed as one member, that one constituent element is formed by a plurality of members, a certain constituent element is a portion of another constituent element, a portion of a certain constituent element and a portion of another constituent element overlap each other, and the like.

Terms in the invention or terms to be used when describing the invention are defined as follows unless otherwise stated.

When describing the invention, the terms "distal end", "distal end part", and "proximal end part" may be appropriately used. Here, the "distal end" means an insertion tip of a medical device body, and the "distal end part" means a predetermined length region including the distal end. Additionally, the "proximal end part" means a predetermined length region including an end part (proximal end) on a base end side of the medical device body. In the invention, an axial center means a central axis in a longitudinal direction of the medical device body.

In the present specification, a "longitudinal section" of the medical device means a section in which the medical device (a catheter in the embodiment) is cut parallel to the longitudinal direction through the axial center.

"Resin hardness" means the physical property value of a resin member itself. For example, the resin hardness of a first resin member or a second resin member can be measured according to well-known test methods, such as the Shore hardness. In addition, in a case where additives other than resin, such as a contrast medium and arbitrary fillers, are contained in the first resin member or the second resin member, the above resin hardness means the resin hardness of the first resin member or the second resin member in which these additives are contained.

### <First Embodiment>

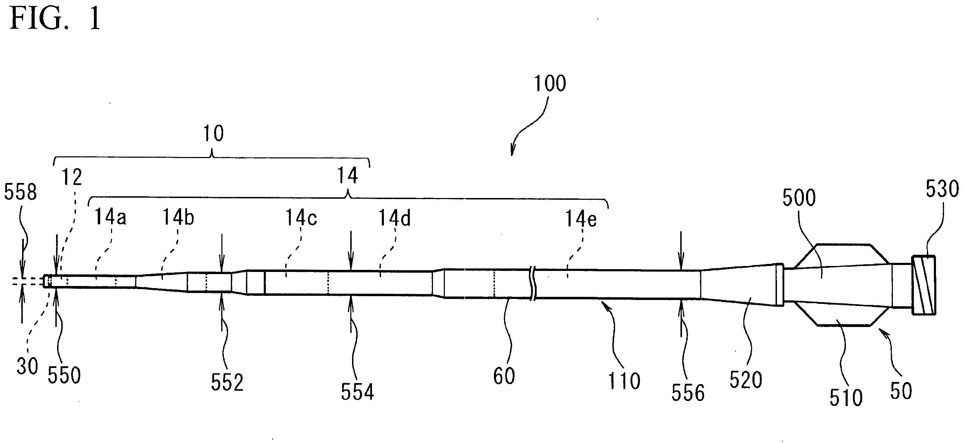

The configuration of a catheter 100 that relates to the medical device of the invention and is a first embodiment of the invention will be described below with reference to FIGS. 1 to 3.

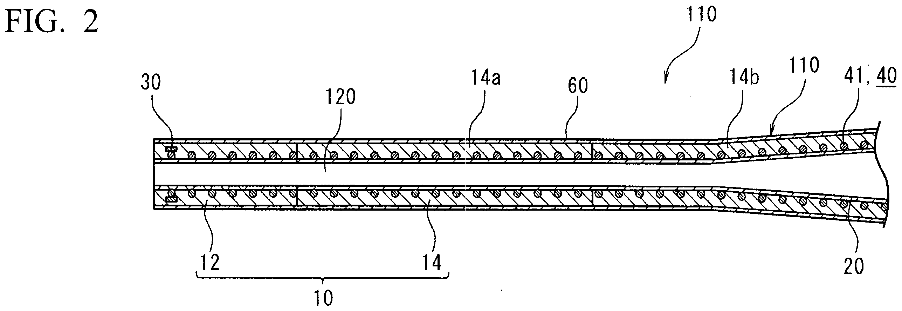

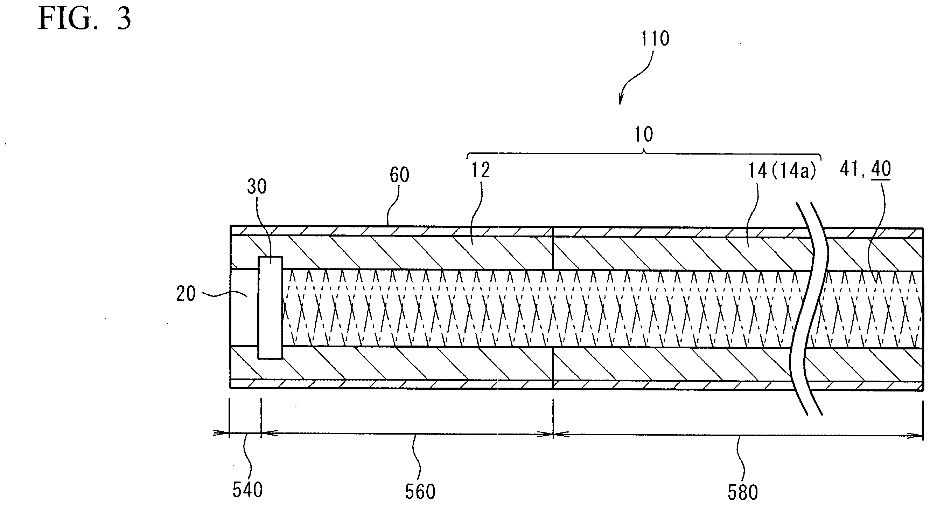

FIG. 1 is an overall view of the catheter 100 that is the medical device in the first embodiment of the invention. FIG. 2 is a longitudinal sectional view of a tip part of the catheter 100. FIG. 3 is an explanatory view of the tip part of a catheter body 110 that is the first embodiment. FIG. 3 is an enlarged view of the tip part illustrated in FIG. 2. FIG. 3 is a sectional view of an outer tube 10 and an outer layer 60 and a side view of an inner tube 20, a marker 30, and a metal protective layer 40.

The medical device (catheter 100) related to the present embodiment is elongated, has flexibility, and has a medical device body (catheter body 110) to be inserted into a body cavity. The medical device body (catheter body 110) has an elongated resin tube (outer tube 10). The resin tube (outer tube 10) has a first region 12 formed of a first resin member, and a second region 14 formed of a second resin member different from the first resin member and is closer to a proximal side than to the first region 12.

In the medical device (catheter 100), a difference X between the resin hardness of the first resin member measured at normal temperature and the resin hardness of the first resin member measured after being left for the predetermined time in a temperature environment of 38°C is greater than a difference Y between the resin hardness of the second resin member measured at the normal temperature, and the resin hardness of the second resin member measured after being left for the predetermined time in the temperature environment of 38°C.

By virtue of this configuration, the outer tube 10 can obtain such a degree of rigidity that exhibits excellent pushability is exhibited when the catheter body also including the first region 12 is inserted into a body cavity. While being exposed to the body temperature (that is, the temperature of about 38°C) for a while in a body cavity, the first region 12 in the outer tube 10 is markedly made flexible, a tip part of the catheter body 110 becomes flexible, and thereby, the branch selectivity of the catheter 100 is improved.

It is greatly advantageous in terms of the operation of the catheter 100 that the branch selectivity is improved in a place where branch selection is difficult and a substantial time is required for a selection operation or in a body cavity terminal region (for example, a blood vessel terminal region) places that is reached over a predetermined-time.

Since the first resin member and the second resin member are different resin members, the above-described different temperature characteristics can be exhibited. Here, the different temperature characteristics include an aspect in which the first resin member and the second resin member are made of different kinds of resin, respectively, an aspect in which one resin member is made of one kind of resin and the other resin member is made of a composition including a plurality of different kinds of resin, and an aspect in which the first resin member and the second resin member are respectively made of compositions including a plurality of different kinds of resin. For example, the first resin member and the second resin member may be made of resin compositions in which two or greater resin components to be contained are the same as each other and component ratios are different from each other.

For example, when the catheter 100 is made to invade within a blood vessel from a predetermined place and is made to invade even a desired living body tissue, the operation of branch selection requires a predetermined time in a case where the catheter has reaches a place where the branch selection is difficult. In this case, the resin hardness of the first resin that constitutes the first region 12 decreases markedly as compared to a resin hardness in a normal temperature environment during lapse of the operation time. That is, the catheter body 110 is made flexible and the branch selectivity of the catheter 100 is improved.

Hereinafter, the medical device applied to the present embodiment will be described in detail.

The medical device (hereinafter refereed to as the catheter 100) related to the present embodiment includes the medical device body (hereinafter referred to as the catheter body 110). The catheter body 110 is elongated, has flexibility, and is inserted into a body cavity. The catheter body 110 has the outer tube 10 that is an elongated resin tube, and the inner tube 20 that is stacked on an inner peripheral side of the outer tube 10. The metal protective layer 40 formed by a wire 41 being wound at predetermined intervals is provided at an outer periphery of the inner tube 20.

The catheter 100 is an example in which an intravascular catheter used after being inserted through a blood vessel is preferable.

The catheter 100 related to the present embodiment is, for example, an inactive catheter. That is, the catheter 100 can be used as an inactive catheter including the catheter body 110 to be inserted into a body cavity by a technique called an over-the-wire using a guide wire, and does not have an operating mechanism that bends the tip part.

As described above, the catheter 100 has excellent followability with respect to a guide wire because the first region 12 is made more flexible than the second region 14 when a predetermined time has lapsed in a body cavity.

The outer tube 10 is an elongated hollow resin tube. As described above, the outer tube 10 includes the first region 12 and the second region 14.

In the present embodiment, the first region 12 constitutes a tip part (distal end part) of the outer tube 10. Additionally, the second region 14 constitutes a base end part (proximal end part) of the outer tube 10. The tip part of the outer tube 10 is a predetermined region including a tip of the outer tube 10 on a distal side of the catheter body 110, or a predetermined region near the tip that does not include the tip. Additionally, in the present embodiment, the base end part of the outer tube 10 is a region closer to a proximal side of the catheter body 110 than to the above tip part, and is a predetermined region including a base end of the outer tube 10 on the proximal side of the catheter body 110 or a predetermined region near the base end that does not include the base end.

In the present embodiment, specifically, as illustrated in FIGS. 2 and 3, the first region 12 that is the tip part of the outer tube 10 is a predetermined region including the tip of the outer tube 10 on the distal side of the catheter body 110.

That is, in the medical device (catheter 100) related to the present embodiment, a tip of the first region 12 constitutes a tip of the resin tube (outer tube 10).

Therefore, in a case where time is significantly required for the operation of branch selection, making the first region 12 flexible excellently contributes to making the tip part of the catheter body 110 flexible.

The first region 12 is formed of the first resin member.

The first resin member includes one or greater kinds of resin. That is, the first resin member may be formed of only a first resin, or may be made of a resin composition in which the first resin and one or greater kinds of other resin other than the first resin are blended together.

The second region 14 is formed of the second resin member.

The second resin member includes one or greater kinds of resin. That is, the second resin member may be formed of only a second resin, or may be made of a resin composition in which the second resin and one or greater kinds of other resin other than the second resin are compounded together.

The first resin and the second resin are different kinds of resin.

In addition, the first resin member that constitutes the first region 12 and the second resin member that constitutes the second region 14 may be appropriately made to contain arbitrary compounding agents as components other than the resin. For example, in order to check the position of the catheter body 110, the first region 12 and/or the second region 14 may be made to contain suitable amounts of contrast media. Additionally, the first region 12 and/or second region 14 may be made to contain pigments for coloring in desired colors, and the above compounding agents are not limited to these kinds of illustration.

The above first resin and the above second resin, or other resin in which these first and second resins are compounded together is, for example, a thermoplastic polymer. This thermoplastic polymer may include polyurethane (PU)-based resin, polyimide (PI)-based resin, polyamide imide (PAI)-based resin, polyethylene terephthalate (PET)-based resin, polyethylene (PE)-based resin, polyamide (PA)-based resin, nylon elastomer-based resin, ethylene-vinyl acetate-based resin (EVA), polyvinyl chloride (PVC)-based resin, or polypropylene (PP)-based resin, or combinations thereof.

The above thermoplastic polymer may include a thermoplastic elastomer.

The thermoplastic elastomer in the present specification is a concept also including a polymer alloy (a polymer blend, a block copolymer, a graft copolymer, a random copolymer, or the like), a substance made flexible with a plasticizer or the like, or mixtures thereof. The above elastomer may include an urethane-based elastomer, an amide-based elastomer (nylon-based elastomer), a styrene-based elastomer, an olefin-based elastomer, or a vinyl chloride-based elastomer. The urethane-based elastomer is, for example, a thermoplastic polyurethane elastomer or the like. The above amide-based elastomer may include a thermoplastic polyamide elastomer or a thermoplastic polyether block amide copolymer.

Although the resin hardness of the first resin member and the second resin member is not particularly limited, it is preferable to have a resin hardness such that excellent pushability can be given to the outer tube 10 in the normal temperature environment. Although the normal temperature is not a strictly specified temperature, the normal temperature is, for example, about 23°C±2°C.

In the catheter 100, a difference between the resin hardness of the first resin member measured in the normal temperature environment and the resin hardness of the first resin member measured after being left for a predetermined time in the temperature environment of 38°C is defined as a difference X. Additionally, a difference between the resin hardness of the second resin member measured in the normal temperature environment and the resin hardness of the second resin member measured after being left for a predetermined time in the temperature environment of 38°C is defined as a difference Y. The difference X is greater than the difference Y.

For example, the difference X is obtained by measuring the Shore hardness (hardness A-1) measured in the normal temperature environment according to ISO868, using a first test piece made of the first resin member cut with a suitable size. Next, the above first test piece is immersed for a predetermined time in a constant temperature bath that is kept warm at 38°C, and the Shore hardness (hardness A-2) measured according to ISO868 is measured. A value obtained by subtracting (hardness A-2) from (hardness A-1) is the difference X.

The difference Y can be obtained by obtaining (hardness B-1) by the same method as (hardness A-1), using a second test piece made of a second resin member cut with the same size as the first test piece, obtaining (hardness B-2) by the same method as (hardness A-2), and subtracting (hardness B-2) from (hardness B-1).

In addition, a method of measuring the Shore hardness of the first resin member and the second resin member as described above is appropriately referred to for measurement of the resin hardness of the first resin member and the second resin member under the normal temperature environment or under a body temperature environment, in the present embodiment.

In the above method, although the difference X and the difference Y as resin hardnesses are described, the difference in the first and second regions is not limited to the resin hardness. For example, a difference may be provided in the "bending strength" described in Example to be described below. This testing method is performed by using a resin member, which constitutes an outer tube in a tip region of a catheter, as a test piece, pressing a tip of the catheter against a load measuring device from a vertical direction under predetermined conditions, and measuring a load. A difference XI or a difference YI can be obtained from a difference between the load of a test piece measured in the normal temperature environment and the load of the test piece measured using the test piece after being left for a predetermined time in the body temperature environment.

When the resin hardness is measured as described above, the predetermined time at which a test piece is left in the temperature environment of 38°C is not particularly limited, and may be adjusted depending on the thickness of the test piece. For example, a test piece may be left in the temperature environment of 38°C until the temperature of the test piece itself reaches 38°C.

The relationship between the first resin member and the second resin member may be adjusted, for example, so that the ratio of the resin hardness of the first resin at the normal temperature to the difference X becomes greater than the ratio of the resin hardness of the second resin at the normal temperature to the difference Y.

In the present embodiment, it is preferable that the first resin member contains 50% or more of the first resin, and the second resin member contains 50% or more of the second resin that is resin different from the first resin.

By configuring the invention so that the physical properties of the first resin member and the second resin member are governed by different kinds of reference materials, respectively, the magnitude relationship between the difference X and the difference Y as described above can be markedly realized.

In the catheter 100 in which the above magnitude relationship is markedly realized, an operator can feel that the branch selectivity is more excellent than, for example, that when the outer tube 10 is operated by the over-the-tube technique, using a catheter of a related-art example formed of only the second resin member.

An example of a more preferable aspect includes the first resin being thermoplastic polyurethane-based resin and the second resin being polyamide-based resin. Such combination excellently solves the problems of the invention, and can realize excellent pushability and excellent branch selectivity.

More specifically, for example, a thermoplastic polyurethane-based elastomer can be selected as the first resin, and a polyamide-based elastomer (nylon-based elastomer) can be selected as the second resin. The first resin member and the second resin member may be formed by compounding these elastomers with other resin, respectively. Additionally, the resin material of the first resin member may be made of only the thermoplastic polyurethane-based elastomer, and the resin material of the second resin member may be made of the polyamide-based elastomer.

Although a specific example of the thermoplastic polyurethane elastomer that is the above first resin may includes "Pellethane" made by Dow Chemical Japan Limited, "Tecothane" or "Tecoflex" made by Lubrizol, or the like, the invention is not limited to this.

Although a specific example of the polyamide-based elastomer that is the above second resin may include "PEBAX" that hat is a commercial item provided by Tokyo Zairyo Co., Ltd., the invention is not limited to this.

In the catheter 100 related to the present embodiment, the first resin member and the second resin member may be constituted so as to satisfy the following (I) and (II).
(I) The resin hardness of the first resin member measured at the normal temperature is equal to or higher than the resin hardness of the second resin member.
(II) The resin hardness of the first resin member measured after being left for the predetermined time at the temperature environment of 38°C is lower than the resin hardness of the second resin member.

That is, at the time of body cavity invasion or before a predetermined time lapses after invasion into a body cavity, the first region 12 formed of the second resin member is made to have a hardness equal to or higher than the second region 14 formed of the first resin member, and contributes to excellent pushability of the tip part of the catheter 100. When the operation time is prolonged in a body cavity and a predetermined time has lapsed, the first region 12 becomes flexible than the second region 14. Accordingly, in the catheter 100, excellent pushability can be maintained at the base end part including the second region 14, and excellent branch selectivity can be exhibited at the tip part including the first region 12.

The first region 12 in the present embodiment is a tip chip locally provided at the tip part of the outer tube 10. For example, the dimension of the first region 12 in a direction of a central axis is 8 mm or greater to 12 mm or smaller and more preferably 5 mm or greater to 15 mm or smaller. By making the dimension of the first region 12 being present in the above range, when a predetermined time lapses in the temperature environment of 38°C and the resin hardness of the first resin member has decrease, excellent branch selectivity is applied to the catheter 100, and it is sufficiently avoided that pushability is impaired.

It is preferable that the second region 14 in the present embodiment is configured such that the rigidity thereof become continuously greater from the distal side toward the proximal side. Specifically, as illustrated in FIG. 1, in the second region 14, a resin member 14a, a resin member 14b, a resin member 14c, a resin member 14d, and a resin member 14e are arranged in this order from the distal side. The resin member 14a, the resin member 14b, the resin member 14c, the resin member 14d, and the resin member 14e are located on an inner peripheral side of the outer layer 60, and boundaries therebetween are illustrated by dashed lines.

In this way, in a case where the second region 14 is configured such that the plurality of resin members are arrayed in the direction of the central axis, the second resin member related to the various features of the invention and compared to the first resin member is a resin member arranged closest to at least the first resin member among the plurality of resin members that constitute the second region 14.

A magnitude relationship between the resin hardnesses (Shore hardnesess), at the normal temperature, of the respective resin members that constitute the second region 14 is the resin member 14a < the resin member 14b < the resin member 14c < the resin member 14d < the resin member 14e.

For example, in a case where all of the resin member 14a, the resin member 14b, the resin member 14c, the resin member 14d, and the resin member 14e are the same kind of resin (for example, all are the polyamide-based elastomer) of which the hardness is adjusted, the resin hardnesses (Shore hardnesses) thereof at 38°C are also similar to the tendency of the magnitude relationship between the resin hardnesses at the normal temperature as described above.

Although not illustrated, as a modification example of the present embodiment, the second region 14 can also formed of one resin member of which the hardness does not vary.

The catheter 100 is provided with the marker 30 for checking a body cavity invasion position of the catheter body 110 with X rays. The marker 30 has a ring shape including a material through which radiation, such as X rays, is not transmitted. Specifically, the marker 30 is formed of a metallic material, such as platinum. The marker 30 is provided, for example, around the inner tube 20.

The marker 30 in the present embodiment is provided at a distal end part of the catheter 100, and is configured to include an X-rays impermeable metallic material. As illustrated in FIGS. 2 and 3, the marker 30 is covered with the first resin member. The dimension of the first region 12 in the direction of the central axis exceeds the width dimension of the marker 30, and can cover the overall marker 30 attached to the inner tube 20 from an outer peripheral side. In the first region 12, by providing a marker 30 in a bending region of the tip of the catheter 100, a bending state of the tip of the catheter 100 can be precisely checked.

Next, the inner tube 20 will be described. As illustrated in FIGS. 2 and 3, the catheter body 110 has the inner tube 20 stacked on an inner periphery of the resin tube (outer tube 10). The inner tube 20 is formed of a tubular resin material. An inner hole 120 is formed at the center of the inner tube 20 (refer to FIG. 2). The inner hole 120 is formed from an end part on the proximal side of the catheter body 110 referred to as a so-called main lumen to an end part on a distal side thereof. A treatment instrument or a guide wire can be appropriately inserted through the inner hole 120, liquids, such as various kinds of medicine or a contrast medium, to be injected into a body cavity can be made to flow through the inner hole, or liquids, such as blood, can be suctioned from the inside of the body.

In the catheter 100, it is preferable to locate a tip of the inner tube 20 at the same position as the tip of the first region 12 in the direction of the central axis. Here, the same position means substantially the same position in the direction of the central axis, and includes a position such that being the same position can be visually checked even if slightly shifted in terms of measurement. For example, the distance between the tip of the inner tube 20 and the tip of the first region 12 is about 0 mm ± 1 mm in the direction of the central axis.

The following effects are exhibited by arranging the inner tube 20 in a substantially whole region of a boundary between the inner hole 120 and the first region 12 (outer tube 10). That is, even in a case where the viscosity of a peripheral surface of the first region 12 has increased when the resin hardness of the first resin member that constitutes the first region 12 decreases in a body cavity, the first region 12 can be prevented from coming into close contact with a treatment instrument or a guide wire that slides on the inner hole 120. In this viewpoint, it is preferable that the inner tube 20 is formed of materials, such as a fluorine-based thermoplastic polymer material, having excellent slidability with respect to medical devices or guide wires.

It is preferable that the material of the inner tube 20 is, for example, a fluorine-based thermoplastic polymer material. Specifically, this fluorine-based thermoplastic polymer material is, for example, polytetrafluoroethylene (PTFE), polyvinylidene fluoride (PVDF), or perfluoroalkoxy fluororesin (PFA).

By constituting the inner tube 20 of such fluorine-based resin, the delivery performance when supplying a contrast medium, a medicinal solution, or the like to living body tissue through the inner hole 120 becomes excellent.

The inner tube 20 in the present embodiment is formed of a third resin member that is a resin member different from the first resin member and the second resin member. For example, as the resin hardness of the third resin member, a resin hardness exceeding the resin hardness of the first resin member and the second resin member in temperature environments of the normal temperature and 38°C can be selected.

However, the invention does not exclude that the outer tube 10 and the inner tube 20 are formed of the same kind of resin member. For example, the invention does not exclude that the third resin member that constitutes the inner tube 20 and either the first resin member or the second resin member are the same kind of resin.

In the catheter 100 in the present embodiment, as a modification example of which the illustration is omitted, a sublumen may be formed inside the outer tube 10. For example, a hollow resin tube having a smaller internal diameter than the internal diameter of the inner hole 120 may be buried inside the outer tube 10, and one or greater sublumens that communicates with the inside of the outer tube 10 from the proximal side to the distal side may be provided. For example, an operating wire that operates the orientation of the tip of the catheter 100 can be inserted through the inside of the above sublumen.

Next, the metal protective layer 40 will be described.

The catheter 100 has the metal protective layer 40 provided on the inner peripheral side or the inside of the resin tube (outer tube 10). Here, the "being provided on the inner peripheral side" means that the metal protective layer 40 is provided in direct or indirect contact with an inner peripheral surface of the outer tube 10. Additionally, the metal protective layer 40 is provided in a state where at least a portion thereof is bitten in a thickness direction of the outer tube 10. FIG. 2 illustrated an aspect in which the metal protective layer 40 is bitten in the thickness direction from the inner peripheral surface side of the outer tube 10. A tip of the metal protective layer 40 is closer to the proximal side than to the tip of the first region 12 in the direction of the central axis. For this reason, the tip of the metal protective layer 40 is prevented from damaging a body cavity. In addition, in FIG. 3, the wire 41 is illustrated in a line of which the thickness is ignored for simplification of illustration.

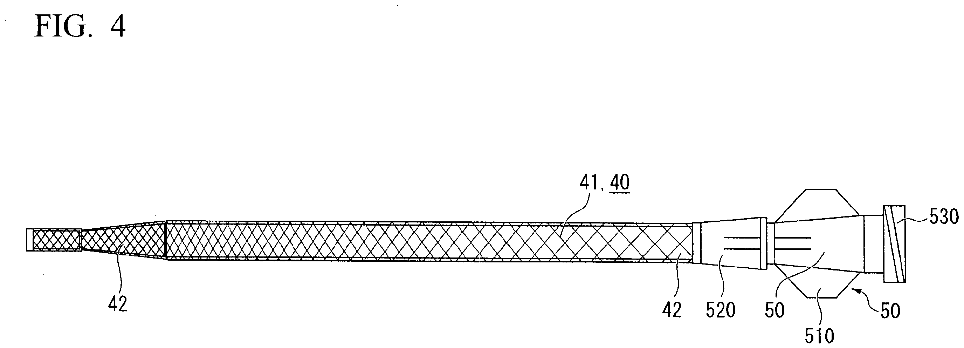

The metal protective layer 40 in the present embodiment adheres to, for example, the inner tube 20 and the outer tube 10. The metal protective layer 40 is configured such that the wire 41 is wound around the inner tube 20. In FIG. 2, a section of the wire 41 lined up from the proximal side toward the distal side is illustrated at the outer periphery of the inner tube 20. In FIG. 3, a plurality of (for example, eight) the wires 41 are wound around the outer periphery of the inner tube 20 in a right-hand spiral shape, and a plurality of (for example, eight) the wires 41 are wound around the outer periphery in a left-hand spiral shape. The respective left-hand wound and right-hand wound wires 41 is illustrated as a so-called metal protective layer 40 in which the wires are braided into each other. In this case, the pitch of the plurality of wires 41 wound in the same direction is about 200 µm. Additionally, the pitch may be changed from the proximal side toward the distal side. In a series of the metal protective layer 40, it is preferable to make a pitch in the first region 12 smaller than a pitch in the second region. Particularly, as illustrated in FIG. 4, it is preferable to narrow the pitch 42 of the wires gradually from the second region to the first region. Accordingly, the flexibility of the tip part of the catheter 100 can be further improved.

Although not illustrated, the metal protective layer 40 can be appropriately changed to a coiled aspect in which one or greater wires 41 are wound in one direction.

As the material of the wires 41 that constitute the metal protective layer 40, for example, it is a preferable example to use metal. However, the invention is not limited to this. Other materials (for example, resin or the like) may be used as long as materials having higher rigidity and higher elasticity than the inner tube 20 and the outer tube 10 are provided. Specifically, for example, tungsten, stainless steel (SUS), a nickel titanium-based alloy, steel, titanium, or a copper alloy can be used as the metallic material of the wires 41.

Next, the outer layer 60 will be described. The outer layer 60 is provided on an outer peripheral side of the outer tube 10.

The outer layer 60 in the present embodiment constitutes an outermost layer of the catheter body 110. The outer layer 60 can be formed of, for example, hydrophilic materials. In addition, the outer layer 60 may be formed only in a region extending over a partial length of a distal end part of the catheter body 110, or may be continued and formed over the total length of the catheter 100.

The outer layer 60 has a hydrophilic property, for example by performing molding using hydrophilic resin materials, such as polyvinyl alcohol (PVA) and polyvinyl pyrrolidone.

Instead of forming the outer layer 60 at the outer periphery of the outer tube 10 using arbitrary materials, a desired property may be added by performing arbitrary surface treatment on an outer peripheral surface of the outer tube 10. For example, lubricity may be added to an outer surface of the outer tube 10 by performing immersion processing on the outer peripheral surface of the outer tube 10.

Next, a gripping part 50 provided on the proximal side of the catheter body 110 will be described. As illustrated in FIG. 1, the gripping part 50 is provided adjacent to a base end of the catheter body 110. The gripping part 50 has a coupling part 530 for allowing an injector (syringe) (not illustrated) to be inserted therethrough from the rear. A screw groove is formed at an outer periphery of the coupling part 530 so that a syringe can be fixed. The coupling part 530 is attachable and detachable. A hub 500 is provided at the center of the gripping part 50. A hollow part passing through to the hub in the direction of the central axis is formed inside the hub 500, and the inner tube 20 and the outer tube 10 extend (not illustrated). The hub 500 has two vane parts 510 that extend outward from an outer peripheral surface thereof and face each other via the axial center. By rotating the vane parts 510 about the axial center, torque operation of rotating the overall catheter body 110 around the axis is possible, and the direction of the tip of the catheter body 110 that has invaded a body cavity can be adjusted.

The protector 520 is provided on a tip side of the hub 500, and covers base end parts of the inner tube 20 and the outer tube 10 that invade the inside of the hub 500.

Here, the typical dimensions of the catheter body 110 of the present embodiment will be described.

The external diameter of the catheter body 110 is, for example, as follows. That is, as illustrated in FIG. 1, an external diameter 550 of the first region 12 is about 570 µm or greater to 770 µm or smaller (for example, 670 µm), the external diameter of the resin member 14a that constitutes a tip part of the second region 14 is equal to the external diameter of the first region 12, an external diameter 552 of a tip part of the resin member 14c is about 700 µm or greater to 900 µm or smaller (for example, 800 µm), an external diameter 554 of the tip part of the resin member 14d is about 770 µm or greater to 970 µm or smaller (for example, 870 µm), and an external diameter 556 of the resin member 14e is about 870 µm or greater to 1070 micrometers or smaller (for example, 970 µm). That is, the maximum external diameter of the catheter body 110 is around 1 mm in diameter, and preferably 1 mm or smaller. Accordingly, the catheter body 110 is insertable through blood vessels, such as a celiac artery.

In the present embodiment, the external diameters of the first region 12, a region formed of the resin members 14a, and a region formed of the resin member 14e are uniform, respectively. Meanwhile, the external diameters of regions formed of the resin members 14b to 14d have diameter-enlarged parts that are increased in diameter from the tip toward the base end in intermediate parts in the direction of the central axis respectively.

As illustrated in FIG. 3, in the direction of the central axis, a distance 540 from a tip of the marker 30 to the tip of the catheter body 110 is about 0.5 mm or greater to 1 mm or smaller, a distance 560 from the tip of the marker 30 to a base end of the first region 12 is about 5 mm or greater to 15 mm or smaller (for example, 10 mm), and a distance 580 from a tip of the region formed of the resin member 14a to a base end thereof is about 15 mm or greater to 25 mm or smaller (for example, 20 mm).

Additionally, as illustrated in FIG. 1, a diameter 558 of the inner hole 120 at the distal end part of the catheter body 110 is about 400 µm or greater or 600 µm smaller (for example, 500 µm) in a range smaller than the external diameter 550 of the first region 12. For example, a guide wire of about φ460 µm is insertable through the inner hole 120. The diameter of the inner hole 120 may be uniform from a tip of the inner hole to a base end, or may also be increased in proportion to an increase in the external diameter of the outer tube 10.

The total length of the catheter body 110 is 1,250 mm.

In the tip part of the catheter 100, the thickness of the inner tube 20 can be about 10 µm or greater to 50 µm or smaller, and the thickness of the outer tube 10 can be about 30 µm or greater to 160 µm or smaller. Particularly, in the first region 12, the thickness of the inner tube 20 and the thickness of the outer tube 10 in the above ranges are particularly suitable, and it is a preferable aspect that the thickness of the outer tube 10 is made greater than the thickness of the inner tube 20.

Next, an example of a general method for manufacturing the catheter 100 will be described.

First, a core member having a peripheral surface that conforms to the shape of the inner hole 120 of the catheter body 110 is prepared, and the inner tube 20 is formed along an outer periphery of the core member. Thereafter, the metal protective layer 40 is formed by winding the wires 41 around the outer peripheral surface of the inner tube 20. The winding of the wires 41 may be performed at a constant pitch by a commercial device or the like. Of course, the pitch may be changed on the way.

Next, the marker 30 is formed by fitting a metal ring made of an X-rays impermeable material to the tip part of the metal protective layer 40 and performing caulking processing.

Next, the outer tube 10 is formed by converting the metal protective layer 40. Specifically, a plurality of hollow resin tubes (specifically, six regions of the first region 12 and the region of the resin member 14a to the region of the resin member 14e) molded using resin members suitable for the respective regions of the outer tube 10 are prepared. In a state where these resin tubes are fitted to the inner tube 20 including the metal protective layer 40 in order and the resin tubes adjacent to each other are made to abut against each other or are slightly overlapped with each other, a thermally contractable member is overlappingly covered and thermally pressed from an outer periphery of a resin tube concerned. Accordingly, the respective resin tubes are made flexible and partially melted, and end parts between the resin tubes adjacent to each other are fused and joined together. Simultaneously, the outer tube 10 that adheres to the metal protective layer 40 can be formed. The core member is extracted after the thermally contractable member crimped on the outer periphery of the outer tube 10 is peeled off. Thereafter, the outer layer 60 is formed at the outer periphery of the outer tube 10 using a hydrophilic material. Additionally, surface treatment may be performed so that the outer peripheral surface of the outer tube 10 has a hydrophilic property. Finally, the manufacture of the catheter 100 is completed by attaching the gripping part 50. In addition, the above description is an example of the method for manufacturing the catheter 100, and does not limit the invention at all.

Next, how to use the catheter 100 will simply described taking an over-the-wire technique as an example.

First, the guide wire is inserted through a body cavity (not illustrated). In this case, the catheter body (not illustrated) may be 1 appropriately installed within the body cavity in advance.

Next, the guide wire inserted through the body cavity is inserted through the body cavity up to a suitable position thereof. For example, a tip of the guide wire is made to reach an arbitrary place where the guide wire has pass through one or greater branches. Subsequently, the catheter body 110 is made to invade the body cavity along the guide wire. When the catheter body 110 reaches the vicinity of the tip of the guide wire, the guide wire is made to further invade a deep part of the body cavity if necessary. Next, the catheter body 110 is moved forward. Thus, the distal end of the catheter 100 is made to reach a desired living body tissue. If the tip part of the catheter body 110 reaches the desired living body tissue, a medicinal solution or a contrast medium is injected into the tissue, and treatment or inspection is performed. Additionally, in order to check the distal end of the catheter 100 while the catheter body 110 is made to invade the body cavity, the guide wire may be pulled out once, the contrast medium may be injected into the inner hole 120 via the coupling part 530 and may be discharged into the body cavity from the distal end of the catheter 100, and positional checking be performed with X rays.

In addition, the first region 12 of the catheter body 110 are as described above, and are markedly made flexible when a predetermined time lapses in the temperature environment (that is, an environment within the body cavity) of 38°C. Therefore, in a branch where selection is difficult, while a predetermined time is required for the operation of bending the catheter body 110 along the guide wire and performing branch selection of the catheter body, the first region 12 is naturally made flexible and the flexibility of the distal end part of the catheter body 110 is increased. Therefore, the branch selectivity of the catheter 100 is improved.

When the first region 12 once made flexible in the body cavity is again made hard so as to improve the pushability of the tip of the catheter body 110, the first region 12 may be cooled by circulating a liquid lower than 38°C (for example, the normal temperature) through the inner hole 120. Otherwise, the catheter body 110 may be extracted and the tip part including the first region 12 may be exposed to the normal temperature or the like, in a state where the guide wire is left in the body cavity.

In addition, the body cavity in the present specification includes any ones if the body cavity is a fine hole inside the body. For example, the body cavity is also applicable to "vessels" in the living body, such as blood vessels or ureters and alimentary canals other than the blood vessels.

Additionally, since the medical device (for example, the catheter 100) of the invention includes the first region and the second region as described above, the flexibility of the tip part of the medical device including the first region inside the body is excellent.

The first region and the second region in the invention may have the following characteristics by reflecting the properties of the first region and the second region as described above.

That is, the difference XI between the bending strength of the above first region measured at the normal temperature and the bending strength of the above first region measured after being left for a predetermined time in the temperature environment of 38°C is greater than the difference YI between the bending strength of the above second region measured at normal temperature and the bending strength of the above second region measured after being left for a predetermined time in the temperature environment of 38°C.

Here, the bending strength of the first region and the second region can be evaluated in a so-called three-point bending test in which bending characteristics are evaluated. More specifically, for example, the first region is taken out by cutting the catheter 100 perpendicularly to the direction of the central axis at the positions of the tip and the base end of the first region. Then, a test piece is made by the taken-out first region being cut in from an outer periphery thereof to the vicinity of the axial center parallel to the direction of the central axis and being deployed in a circumferential direction, and the three-point bending test is performed using the test piece. In the three-point bending test, fulcrums are applied to a tip side and a base end side of the first region, and, a load is applied to an intermediate part therebetween. The bending strength of the second region can also be tested similarly. However, in order to arrange the preparation conditions of the first region and the first test piece to be compared with each other, a test piece of a second region may be made by the second region being cut off from a tip (that is, a boundary position between the first region and the second region) of the second region to a position equivalent to the length of the first region in the direction of the central axis, being cut in up to the vicinity of the axial center, and being deployed in the circumferential direction.

Additionally, a different test in which the above difference XI and the above difference YI are compared with each other may include the following method. That is, a catheter specimen having the same configuration as the first region as the configuration of a catheter body and a catheter specimen having the same configuration as the second region as the configuration of a catheter body are respectively made, and these catheter specimens are tested by the same method as a tip bending load test illustrated in Example to be described below. Accordingly, the decreasing rate of the bending strength of the first region and the decreasing rate of the bending strength of the second region in the normal temperature environment and the body temperature environment can be estimated.

### <Second Embodiment>

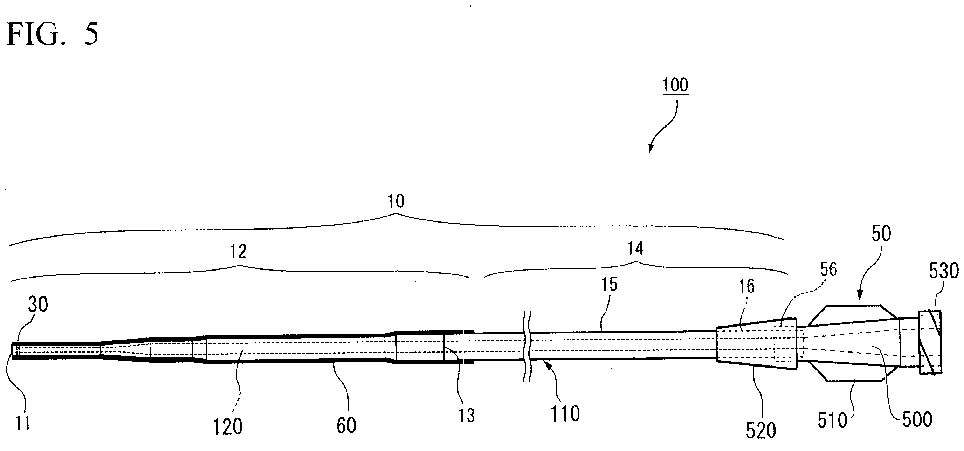

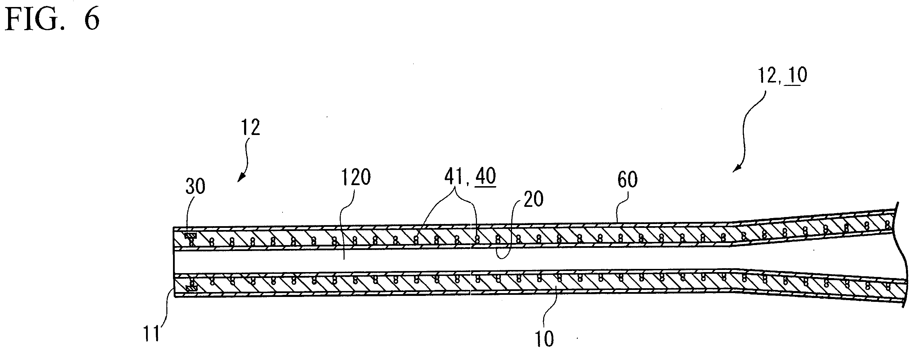

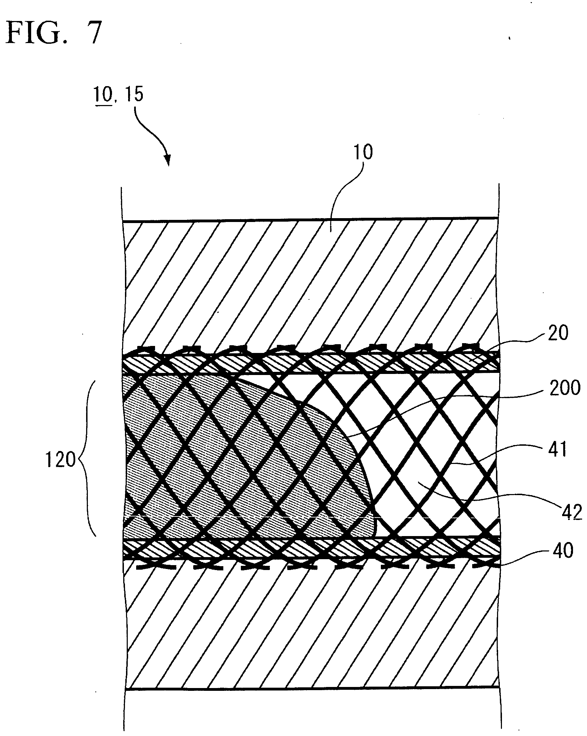

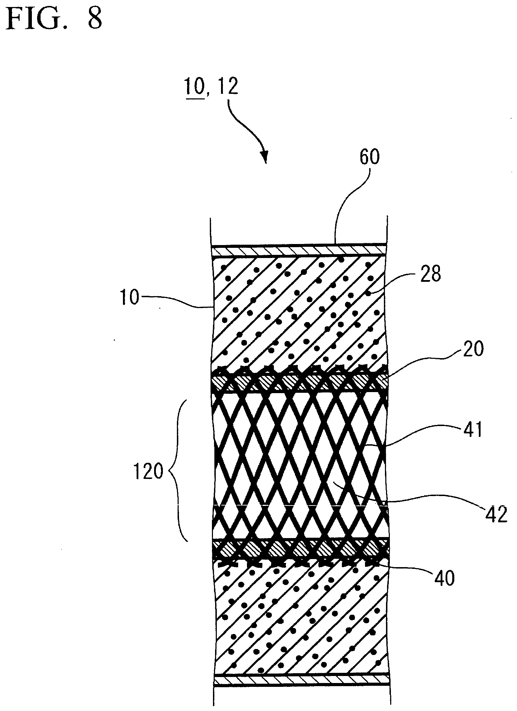

FIG. 5 is a side view illustrating the catheter 100 that is another example of the medical device related to the embodiment of the invention, and a partial length region of the second region 14 of the catheter body 110 is not illustrated. FIG. 6 is a longitudinal sectional view illustrating the internal structure of the first region 12 of the catheter body 110, and a portion of the first region 12 on the base end side is not illustrated. FIG. 7 is an enlarged view schematically illustrating an extending region 15 in the second region 14 of the catheter body 110, and FIG. 8 is an enlarged view schematically illustrating the first region 12.

First, the outline of the medical device of the present embodiment will be described.

The medical device (catheter) 100 of the present embodiment includes has the catheter body 110 having an elongated tubular shape and having the inner hole 120, a hollow hub 500, and the protector 520.

The hub 500 is provided on a base end side of the catheter body 110 so as to communicate with the inner hole 120 of the catheter body 110. The protector 520 is provided at a tip part of the hub 500, and holds an outer periphery of the second region 14 of the catheter body 110.

In the medical device (catheter) 100 of the present embodiment, both the protector 520 and a holding region 16 held by the protector 520 in the second region 14 of the catheter body 110 have visible light permeability, and are the internal state of the inner hole 120 is visually recognizable (refer to FIG. 7).

Here, the "being capable of visually recognizing the internal state of the inner hole 120" means that, when a colored passing object 200, such as blood, is made to pass through the inner hole 120, the passage of the passing object 200 can be visually recognized in an ordinary procedure environment.

Next, the medical device of the present embodiment will be described in detail.

The medical device of the present embodiment is, for example, the catheter 100. More specifically, the medical device is an inactive catheter that does not have an operating mechanism that bends the distal end part of the catheter body 110. The medical device various catheters to be used after being inserted into various bodies cavities, such as an abdominal cavity, the thorax, or a ureter, in addition to an intravascular catheter or a digestive organ catheter. Among these, the catheter 100 of the present embodiment is suitably used particularly for an intravascular catheter from a viewpoint that blood (reverse flow blood) that flows backwards from a target living body tissue to the inner hole 120 of the catheter 100 can be visually checked.

In addition, the invention is applicable to various kinds of elongated medical devices to be used after being inserted into forceps hole of an endoscope. As the medical device, a puncturing needle for an endoscope (local injection needle) can be exemplified. The puncturing needle for an endoscope is an instrument in which an inner tube having a puncturing needle provided at a distal end part thereof is slidably inserted through a larger-diameter outer tube than this inner tube, a syringe is attached to a base end of the inner tube and, and liquids, such as a medicinal solution, are discharged from the puncturing needle to a target living body tissue.

The catheter body 110 is elongated and hollow, and has one or a plurality inner holes 120 therein. The catheter body 110 has a length of about several tens of centimeters to several meters. In the present embodiment, the catheter body 110 is divided roughly into the first region 12 and the second region 14, which will be described. The first region 12 is a length region of about several millimeters to 1 meter, and the second region 14 is the remaining length region of the catheter body 110.

A gripping part that users, such as a doctor, grip with his/her fingers is provided on the base end side of the catheter body 110. The coupling part 530 is provided at a proximal end of the hub 500. The inside of the coupling part 530 communicates with the inner hole 120 and opens thereto. A spiral groove is formed at an outer periphery of the coupling part 530, and has a lure connector of a syringe (not illustrated) threadedly mounted thereon. Accordingly, medicines such as a medicinal solution or embolismic beads, with which the syringe is filled, can be supplied to the catheter body 110. Additionally, a body fluid, a blocker, or the like can also be suctioned from the inside of a body cavity through the catheter body 110. Moreover, instruments, such as a guide wire or a stent, may be inserted into an opening of the coupling part 530.

A plurality of vane parts 510 are formed around the hub 500. By rotating the vane parts 510 about an axial center of the hub 500, the torque for rotating the catheter body 110 around the axils center can be applied. Accordingly, the catheter body 110 inserted into a body cavity can be directed to a desired direction.

The hub 500 is made of a resin material. Polyamide-based resin (nylon) or polyolefin can be exemplified as the resin material. The hub 500 has visible light permeability. Accordingly, it can be visually checked that the insertion depth of the tip of the syringe is normal. Additionally, it can be visually check that medicine is normally discharged from the syringe in the hub 500.

A coupling recess 56 is formed at the tip part of the hub 500, and a proximal end of the catheter body 110 is fitted and fixed to the coupling recess 56. The coupling recess 56 is present at the boundary between the catheter body 110 and the hub 500.

The protector 520 is a hollow tubular member provided at the tip part of the hub 500. A portion (holding region 16) of the second region 14 of the catheter body 110 is inserted through the protector 520. As illustrated in FIG. 5, a portion, on the base end side, of the coupling recess 56 of the hub 500 is exposed slightly closer to the proximal side than the protector 520. Accordingly, a boundary between the catheter body 110 and the gripping part 50 can be visually recognized clearly through the hub 500 without via the protector 520. However, the invention is not limited to this, and the overall coupling recess 56 of the hub 500 may be covered with the protector 520. The protector 520 has a predetermined bending rigidity, and functions as a strain reliever that relieves a bending stress concentrated on a proximal-most portion in the vicinity of the hub 500 in the catheter body 110 to prevent the kink of the catheter body 110.

In the catheter 100 of the present embodiment, both of the protector 520 and the holding regions 16 of the catheter body 110 have visible light permeability.

The "catheter body 110 has visible light permeability" means that the catheter body 110 is transparent or translucent and visible light is transmitted through the catheter body from the outside of the catheter body 110 to the inner hole 120 such that the internal state of the inner hole 120 can be visually recognized. In a case where the catheter body 110 has a multilayer structure, the "catheter body 110 has visible light permeability" means that visible light is transmitted through the multiple structure as described above over the overall multilayer structure. Additionally, for example, in a case where the medical device is a puncturing needle for an endoscope (local injection needle), the catheter body 110 may include a duplex (multiplex) tube structure in which an inner tube is slidably inserted through the inside of an outer tube. In this case, the "the catheter body 110 has visible light permeability" means that, in a composite state where an outer tube and an inner tube are combined together, visible light is transmitted through the catheter body at least from the outside of the catheter body 110 to the inner hole 120 of the inner tube, so that the inside of the catheter body can be visually recognized.

Additionally, the "protector 520 has visible light permeability" means that the protector 520 is transparent or translucent and visible light is transmitted through the protector from the outside of the protector 520 to the inside thereof such that the internal state of the hollow tubular protector 520 can be visually recognized. In a case where the protector 520 has a multilayer structure, the "protector 520 has visible light permeability" means that visible light is transmitted through the multiple structure as described above over the overall multilayer structure.

In addition, also in the present embodiment, the catheter body 110 has the outer tube 10 formed of resin, similar to the above first embodiment. The outer tube 10 has a first region including a distal-side end part and a second region including a base-end-side end part. The first region is more flexible than the above second region.

As described above, since the medical device of the present embodiment has the same configuration as the above first embodiment, branch selectivity is excellent while excellent pushability in extremely narrow body cavities, such as an intricately curved body cavity or a blood vessel terminal region, is maintained. Moreover, according to the catheter 100 of the present embodiment, medicine that flows through the catheter body 110, or reverse flow blood that has reached the protector 520 from the distal end 11 through the inner hole 120 of the catheter body 110 can be visually checked from the outside. For this reason, it can be visually checked that medicine flows normally toward the tip side of the catheter body 110 without becoming blocked by the coupling recess 56 that is the boundary between the catheter body 110 and the hub 500. Additionally, reverse flow blood can be visually checked immediately, at the latest when the reverse flow blood has reached the protector 520 before the reverse flow blood reaches an intermediate part of the hub 500 beyond the protector 520.

The visible light transmittance of the second region 14 of the catheter body 110 is preferably 50% or more and more preferably 70% or more, and the visible light transmittance of the protector 520 is preferably 30% or more and more preferably 50% or more. Here, the visible light transmittance of each member means the transmittance of visible light from the outside of the member to an axial center (the inner hole 120 in the case of the catheter body 110) thereof. In the catheter 100 of the present embodiment, when the visible light transmittance of the second region 14 of the catheter body 110 and the visible light transmittance of the protector 520 are within the above ranges, the colored passing object 200, such as reverse flow blood, which passes through the inner hole 120, can be visually recognized from the outside.

The protector 520 of the present embodiment is made of a resin material, and is transparently or translucently configured. As specific resin materials, polyvinyl chloride, polyurethane, and polyamide can be exemplified in addition to styrene-based resin, such as an olefin styrene copolymer; polyolefin, such as polyethylene, polypropylene, and an ethylene-vinyl acetate copolymer; polyester, such as polyethylene terephthalate; and rubber materials, such as silicone rubber. These resin materials can be used by combining one kind or two or more kinds of materials. Particularly, the visible light transmittance of the protector 520 can be adjusted to the above range by mixing and using resin materials having different light transmittances in a predetermined ratio. Additionally, the protector 520 may not contain any pigment substantially, or may also contain a pigment in a range where transparency or translucency is not impaired.

In the present embodiment, the extending region 15 that extended closer to the tip side than to the protector 520 in the second region 14 of the catheter body 110 also has visible light permeability. More specifically, the holding region 16 and the extending region 15 of the catheter body 110 are translucent. The first region 12 closer to the tip side than to the extending region 15 in the catheter body 110 has visible light impermeability.

Accordingly, reverse flow blood can be visually checked immediately before the reverse flow blood reaches the protector 520 when the passing object 200, such as reverse flow blood, reaches the extending region 15 of the catheter body 110 in a state where the extending region 15 is made to protrude from a master catheter or an endoscope (refer to FIG. 7).

As illustrated in FIGS. 6 to 8, the catheter body 110 has a stacked configuration, and is formed by stacking the inner tube 20 and the outer tube 10 sequentially from the axial center side. The metal protective layer 40 is buried inside the outer tube 10. The metal protective layer 40 is in contact with or is proximity to an outer surface of the inner tube 20.

The inner tube 20 is a tubular member having the inner hole 120 therein. As illustrated in FIG. 6, the inner hole 120 is increased in diameter toward the proximal side, in the first region 12 of the catheter body 110.

The material of the inner tube 20 may include a fluorine-based thermoplastic polymer material. The fluorine-based thermoplastic polymer materials can include, specifically, polytetrafluoroethylene (PTFE), polyvinylidene fluoride (PVDF), and perfluoroalkoxy fluororesin (PFA). These resin materials can be used by combining one kind or two or more kinds of materials.

The material of the outer tube 10 may include a thermoplastic polymer material. This thermoplastic polymer may include polyurethane (PU)-based resin, polyimide (PI)-based resin, polyamide imide (PAI)-based resin, polyethylene terephthalate (PET)-based resin, polyethylene (PE)-based resin, polyamide (PA)-based resin, nylon elastomer-based resin, ethylene-vinyl acetate-based resin (EVA), polyvinyl chloride (PVC)-based resin, or polypropylene (PP)-based resin, or combinations thereof. In addition, the above thermoplastic polymer include a thermoplastic elastomer. These resin materials can be used by combining one kind or two or more kinds of materials.

The thermoplastic elastomer in the present specification is a concept also including a polymer alloy (a polymer blend, a block copolymer, a graft copolymer, a random copolymer, or the like), a substance made flexible with a plasticizer or the like, or mixtures thereof. The above elastomer includes an urethane-based elastomer, an amide-based elastomer (nylon-based elastomer), a styrene-based elastomer, an olefin-based elastomer, or a vinyl chloride-based elastomer. The urethane-based elastomer includes a thermoplastic polyurethane elastomer or the like. The above amide-based elastomer includes a thermoplastic polyamide elastomer or a thermoplastic polyether block amide copolymer.

As illustrated in FIG. 8, a pigment 28 is dispersed in the outer tube 10, in the first region 12 of the catheter body 110. Accordingly, the first region 12 of the catheter body 110 has visible light impermeability and has high visibility. That is, if the first region 12 has visible light permeability, since an instrument platform or the like can be seen through the first region 12 in a state where the catheter 100 is placed on the instrument platform or the like, the visibility of the first region 12 becomes low. As a result, the excellent visibility of the first region 12 is secured by making the first region 12 have visible light impermeability as in the present embodiment. Particularly, visibility can be made still higher by making the first region 12 of the catheter body 110 colored.

The marker 30 is provided in the vicinity of the distal end 11 of the catheter body 110. The marker 30 has a ring shape, and is embedded in the outer tube 10 crimped and fixed to the outer periphery of the tip part of the metal protective layer 40. The marker 30 includes a material through which radiation, such as X rays, is not transmitted, and is formed of, specifically, metallic materials, such as platinum.

External diameter is the same over the extending region 15 and the first region 12 of the catheter body 110. That is, the external diameter of the catheter body 110 is continuous in a boundary part 13 between the second region 14 of visible light permeability and the first region 12 of visible light impermeability. In the first region 12 of the catheter body 110, the external diameter of the catheter body decreases toward the distal end 11 of the catheter body 110.

The catheter 100 of the present embodiment is an intravascular catheter used after being inserted into blood vessels, such as a celiac artery. Here, the dimensions of the catheter 100 of the present embodiment will be described.

The external diameter of the first region 12 of the catheter body 110 can be, for example, 550 µm or greater to 1000 µm or smaller. Additionally, the external diameter of the second region 14 of the catheter body 110 can be 800 µm or greater to 1100 µm or smaller. That is, the maximum external diameter of the catheter body 110 is around 1 mm in diameter, and preferably 1 mm or smaller.

As illustrated in FIG. 5, the external diameter (sheath external diameter) of the first region 12 of the catheter body 110 of the present embodiment is reduced in multiple steps (three steps in the present embodiment). The catheter body 110 is continuously and smoothly reduced in the respectively steps.

The internal diameter of the inner hole 120 of the catheter body 110 can be 400 µm or greater to 600 µm or smaller. The inner hole 120 is increased in diameter toward the proximal side in the first region 12, within the above internal diameter range.

As illustrated in FIGS. 5 and 6, the outer layer 60 is provided on the outer surface of the catheter body 110 over the first region 12 to the extending region 15. The outer layer 60 terminates in the middle of the extending region 15 of the catheter body 110. Accordingly, the pushability of the catheter body 110 within a body cavity is secured, and the handlability of the second region 14 of the catheter body 110 becomes excellent.

The outer layer 60 includes, for example, hydrophilic materials, such as polyvinyl alcohol (PVA) and polyvinyl pyrrolidone. These resin materials can be used by combining one kind or two or more kinds of materials. The outer layer 60 may be formed by subjecting the surface of the outer tube 10 to hydrophilic treatment, or may be formed by coating a hydrophilic resin material on the surface of the outer tube 10.

As illustrated in FIG. 5, the outer layer 60 is formed slightly closer to the base end side than the first region 12 of visible light impermeability in the catheter body 110, and terminates closer to the tip side than an intermediate part of the extending region 15. In other words, the outer layer 60 terminates at a position where the boundary part 13 between the first region 12 and the second region 14 (extending region 15) is exceeded slightly toward the base end side. Even if discontinuity has occurred in the surface properties of the outer tube 10 in the boundary part 13 between the first region 12 where a pigment is mixed in the outer tube 10, and the second region 14 where no pigment is substantially mixed, since the outer layer 60 covers the boundary part 13, the discontinuity of sliding resistance of the catheter body 110 does not occur in the boundary part 13.

The metal protective layer 40 is a protective layer for improving the kink resistance of the catheter body 110 and the pressure resistance of the inner tube 20. The metal protective layer 40 is a coil in which one or a plurality of the wires 41 are spirally wound, or a mesh in which a plurality of the wires 41 are braided.

The material of the wires 41 that constitute the metal protective layer 40 has higher rigidity than the inner tube 20 and the outer tube 10, and for example, a metallic material can be used. Specifically, tungsten, stainless steel (SUS), a nickel titanium-based alloy, steel, titanium, or a copper alloy can be used. In addition, resin materials can be used for the wires 41. These resin materials can be used by combining one kind or two or more kinds of materials.

More specifically, the metal protective layer 40 of the present embodiment is a mesh in which the wires 41 are braided. The metal protective layer 40 is provided inside at least the second region 14 of the catheter body 110. The opening area ratio of the metal protective layer 40 in the second region 14 of the catheter body 110 exceeds 50%. The metal protective layer 40 of the present embodiment can be made by winding a plurality of (for example, eight) wires 41 in a right-hand spiral shape around the outer periphery of the inner tube 20, winding a plurality of (for example, eight) wires 41 in a left-hand spiral shape around the outer periphery of the inner tube 20, and braiding the respective wires 41 into each other.

Accordingly, even if the wires 41 is made of a metallic material of visible light impermeability, the overall catheter body 110 can have visible light permeability.

As illustrated in FIGS. 7 and 8, mesh openings 42 of the metal protective layer 40 are gap portions of the wires 41 that intersect each other. The opening area ratio of the metal protective layer 40 is a ratio of the gap portions (mesh openings 42) where the wires 41 are not present, in the surrounding area of the tubular metal protective layer 40. The opening area ratio may be theoretically calculated on the basis of the wire diameter and pitch of the wires 41, or the area of the metal protective layer may be calculated on the basis of an image of the catheter body 110 (metal protective layer 40). The pitch of the wires 41 is an axial distance between the wires 41 that are wound in the same direction and are adjacent to each other.

The metal protective layer 40 of the present embodiment is provided over the first region 12 of the catheter body 110 to the second region 14 thereof. The opening area ratio (base end opening ratio) of the metal protective layer 40 (refer to FIG. 7) in the second region 14 of the catheter body 110 is greater than the opening area ratio (tip opening ratio) of the metal protective layer 40 (refer to FIG. 8) in the first region 12 of the catheter body 110.

By making the base end opening ratio greater, the metal protective layer 40 does not impair the visibility inside the inner hole 120 in the extending region 15 or the holding region 16 of the second region 14 of the catheter body 110. It is preferable that the base end opening ratio is 70% or more and 90% or less. Accordingly, the visible light permeability in the second region 14 of the catheter body 110 can be made high, and the kink resistance of the second region 14 of the catheter body 110 can be secured.

In the catheter 100 of the present embodiment, the metal protective layer 40 is continuously formed by the same number of wires 41 over the first region 12 to the second region 14. However, the pitch of the wires 41 in the first region 12 of the catheter body 110 is smaller than the pitch of the wires 41 in the second region 14. In an intermediate region including the boundary part 13, the pitch of the wires 41 becomes gradually greater toward the base end side.

In this way, by making the pitch of the wires 41 smaller in the first region 12 of the catheter body 110 to make the tip opening ratio smaller, the spring property of the metal protective layer 40 can be made higher. For this reason, the first region 12 of the catheter body 110 can be flexibly bent.. The tip opening ratio is smaller than the base end opening ratio and is preferably 50% or more and 80% or less.

In addition, the invention is not limited to the aforementioned embodiments, and forms, such as various modifications and improvements, will also be included in the invention as long as the object of the invention is achieved.

In addition, the various constituent elements of the invention do not need to become separately independent. The invention allows that a plurality of constituent elements are formed as one member, that one constituent element is formed by a plurality of members, that a certain constituent element is a portion of another constituent element, that a portion of a certain constituent element and a portion of another constituent element overlap each other, or the like.

### [Examples]

### (Example 1)

A catheter having the same configuration as the catheter 100 illustrated in the above-described first embodiment was made. In detail, the diameter 558 of the inner hole 120 in the distal end part of the catheter body 110 was 500 µm, and the external diameter 550 was 670 µm (refer to FIG. 1). That is, the thickness of the outer tube 10 in the first region 12 was 85 µm. Pellethane 80AE (made by Dow Chemical Japan, Inc.) was used as the first resin member that constitutes the first region 12. The catheter made as above was adopted Example 1.

### (Comparative Example 1)

A catheter configured similar to Example 1 except that Pebox 2533 (provided by Tokyo Zairo Co., Ltd.) was used as the first resin member that constitutes the first region 12 and contained 40 mass % of barium sulfate was prepared, and this catheter was adopted as Comparative Example 1.

### (Tip Bending Load Test)

A tip bending load test was performed using Example 1 and Comparative Example 1 as follows.

First, a guide pipe having a hole with an internal diameter of 0.8 mm, which extends in a linear direction and having both end openings with a length of 3 cm was prepared.

An electronic balance was placed so that a measuring surface becomes horizontal. The extending direction of the above hole was made to be a direction perpendicular to the above measuring surface, and the guide pipe was fixed at a height where the distance from a lower end (lower end opening of the hole) of the guide pipe to the measuring surface of the electronic balance reaches 5 mm.

In the temperature environment of the normal temperature (23°C), the tip of Example 1 was made to invade the guide pipe from an upper end of the hole thereof and was exposed from a lower end of the hole. The tip of Example 1 was pressed against the measuring surface of the electronic balance until the exposure length from the lower end reaches 7 mm. Accordingly, a load displayed on the electronic balance was measured as a "normal temperature environmental load". The measurement was performed 5 times.

Also in Comparative Example 1, the normal temperature environmental load was measured similar to Example 1.

Next, the catheter from the tip of Example 1 to about 80 cm was immersed in a constant temperature bath of which the temperature control was measured for 10 minutes to 38°C. The load was measured by the same method as the method of measuring the normal temperature environmental load except that a catheter immediately after being taken out from the constant temperature bath was used, and this load was adopted as a "body temperature environmental load". Also regarding Comparative Example 1, the body temperature environmental load was similarly measured.

Regarding Example 1, an average value of measurement values (N = 3) excluding a maximum value and a minimum value were obtained from the normal temperature environmental load (N = 5) measured as above. Similarly, an average value of the body temperature environmental load was also obtained. As a result, the average value of the normal temperature environmental load of Example 1 was 1.048 (gf), the average value of the body temperature environmental load was 0.730 (gf), and a difference A between the normal temperature environmental load and the body temperature environmental load was 0.318.

Additionally, the average values of the normal temperature environmental load and the body temperature environmental load of Comparative Example 1 were similarly obtained. As a result, the average value of the normal temperature environmental load was 1.212 (gf), the average value of the body temperature environmental load was 1.105 (gf), and a difference B between the normal temperature environmental load and the body temperature environmental load was 0.107.

From the above results, it was confirmed that the difference A in Example 1 is greater than the difference B in Comparative Example 1. That is, Example 1 is greater than Comparative Example 1 in the degree of flexibility the tip region after being heated to 38°C. Since Example 1 and Comparative Example 1 were similarly configured except that the resin members that constitute the first region 12 of the outer tube 10 are different from each other, it was understood that a difference between the difference A and difference B results from the properties of the resin members that were chiefly used.

Additionally, it was estimated from the behaviors of Example 1 and Comparative Example 1 that the difference X in the first region 12 becomes greater than the difference Y in the second region 14, In a case where the thermoplastic urethane-based elastomer was used as the first resin member that constitutes the first region 12 and the amide-based elastomer was used as the second resin member that constitutes the second region 14. That is, it was estimated that, due to being exposed to the temperature environment of 38°C for a predetermined time, the degree of flexibility of the first region 12 becomes greater than that of the second region 14, and the branch selectivity of the catheter is improved.

### Reference Signs List

- 10:: OUTER TUBE (RESIN TUBE)
- 12:: FIRST REGION
- 13:: BOUNDARY PART
- 14:: SECOND REGION
- 14a:: RESIN MEMBER
- 14b:: RESIN MEMBER
- 14c:: RESIN MEMBER
- 14d:: RESIN MEMBER
- 14e:: RESIN MEMBER
- 15:: EXTENDING REGION
- 16:: HOLDING REGION
- 20:: INNER TUBE
- 30:: MARKER
- 40:: METAL PROTECTIVE LAYER
- 41:: WIRE
- 50:: GRIPPING PART
- 52:: COUPLING PART
- 56:: COUPLING RECESS
- 60:: OUTER LAYER
- 100:: CATHETER
- 110:: CATHETER BODY
- 120:: INNER HOLE
- 500:: HUB
- 510:: VANE PART
- 520:: PROTECTOR
- 530:: COUPLING PART
- 540:: DISTANCE
- 550:: EXTERNAL DIAMETER
- 552:: EXTERNAL DIAMETER
- 554:: EXTERNAL DIAMETER
- 556:: EXTERNAL DIAMETER
- 558:: DIAMETER
- 560:: DISTANCE
- 580:: DISTANCE

## Claims

1. A medical device comprising a medical device body to be inserted into a body cavity,
wherein the medical device body includes an elongated resin tube,
wherein the resin tube includes a first region including a distal end part and a second region including a proximal end part, and
wherein the first region is more flexible than the second region.

2. The medical device according to Claim 1,
wherein the first region is formed of a first resin member,
wherein the second region is formed of a resin member different from the first resin member, and
wherein a difference X between a resin hardness of the first resin member measured at normal temperature and a resin hardness of the first resin member measured after being left for a predetermined time in a temperature environment of 38°C is greater than a difference Y between a resin hardness of the second resin member measured at the normal temperature, and the resin hardness of the second resin member measured after being left for the predetermined time in the temperature environment of 38°C.

3. The medical device according to Claim 2,
wherein the first resin member contains 50% or more of a first resin, and the second resin member contains 50% or more of a second resin that is resin different from the first resin.

4. The medical device according to Claim 2 or 3,
wherein the first resin is thermoplastic polyurethane-based resin, and the second resin is polyamide-based resin.

5. The medical device according to any one of Claims 2 to 4,
wherein, in the first resin member and the second resin member,
(I) the resin hardness of the first resin member measured at the normal temperature is greater than the resin hardness of the second resin member, and
(II) the resin hardness of the first resin member measured after being left for the predetermined time in the temperature environment of 38°C is smaller than the resin hardness of the second resin member.

6. The medical device according to any one of Claims 2 to 5,
wherein the medical device further comprises an inner tube stacked on an inner periphery of the resin tube,
wherein a tip of the inner tube is located at the same position as a tip of the first region in a direction of a central axis.

7. The medical device according to any one of Claims 2 to 6,
wherein a tip of the first region constitutes a tip of the resin tube.

8. The medical device according to any one of Claims 2 to 7,
wherein a marker that is provided at a distal end part and includes an X-ray impermeable metallic material is covered with the first resin member.

9. The medical device according to any one of Claims 2 to 8,
wherein the medical device further comprises a metal protective layer provided on an inner peripheral side of the resin tube or inside the resin tube,
wherein a tip of the metal protective layer is closer to a base-end-side end part than to the tip of the first region in a direction of a central axis.

10. The medical device according to any one of Claims 2 to 9,
wherein the dimension of the first region in the direction of the central axis is 5 mm or greater to 15 mm or smaller.

11. The medical device according to Claim 1,
wherein a metal protective layer formed by braiding wires is provided inside at least the second region of the resin tube, and an opening area ratio of the metal protective layer in a base-end-side end part of the resin tube exceeds 50%.

12. The medical device according to Claim 11,
wherein the metal protective layer is provided in the first region and the second region of the resin tube, and
wherein the opening area ratio of the metal protective layer in the base-end-side end part of the second region is greater than the opening area ratio in an insertion-tip-side end part of the first region.

13. The medical device according to Claim 11 or 12,
wherein the pitch of the wires becomes gradually narrower over the second region to the first region.

14. The medical device according to Claim 1,
wherein the medical device further comprises a hollow hub provided at a base-end-side end part of the resin tube so as to communicate with an inner hole of the resin tube and a protector provided at a tip part of the hub to hold an outer periphery of the base-end-side end part of the resin tube,
wherein both the protector and a holding region held by the protector in the base-end-side end part of the resin tube have visible light permeability, and an internal state of the inner hole is visually recognizable.

15. The medical device according to Claim 14,
wherein an extending region that extends closer to a tip side than to the protector in the base-end-side end part of the resin tube has visible light permeability, and a tip part closer to the tip side than to the extending region, in the resin tube, has visible light impermeability.

16. The medical device according to Claim 15,
wherein the holding region and the extending region of the resin tube are translucent.

17. The medical device according to Claim 15 or 16,
wherein the outer diameter of the resin tube is the same over the extending region and the tip part of the resin tube, and the tip part of the resin tube is adapted such that the outer diameter of the resin tube decreases toward a distal end of the resin tube.

18. The medical device according to any one of Claims 15 to 17,
wherein a hydrophilic coat layer is provided on the outer surface of the resin tube over the insertion-tip-side end part to the extending region, and the hydrophilic coat layer terminates in the middle of the extending region.

19. The medical device according to any one of Claims 1 to 18,
wherein the medical device is a catheter.
